# EUROPEAN PATENT APPLICATION

(11) **EP 1 312 674 A1**
(43) Date of publication of application: **21.05.2003**
(21) Application number: 02025704.4
(22) Date of filing: 15.11.2002
(51) Int. Cl.: C12N 15/11

(54) **Allosteric ribozymes and uses thereof**

(30) Priority: 15.11.2001 EP 01127156
(71) Applicant: Noxxon Pharma AG, 10589 Berlin (DE)
(72) Inventor: Klussmann, Sven, 10709 Berlin (DE); Frauendorf, Christian, 13189 Berlin (DE)
(74) Representative: Bohmann, Armin K., Dr.

(57) **Abstract**

The present invention is related to an allosteric (deoxy) ribozyme, wherein the ribozyme consists of L-nucleotides.

SEQUENCE LISTING

<110> NOXXON Pharma AG

<120> Allosteric Ribozymes and uses thereof

<130> N 10036 EP

<160> 5

<170> PatentIn version 3.1

<210> 1
<211> 57
<212> RNA
<213> Unknown

<220>
<223> allosteric ribozyme

<220>
<221> modified_base
<222> (1)..(1)
<223> fluoresceine labelled

<400> 1
gcgucucuga ugagucguau accagccgaa aggcccuugg cagauuccga aacucug 57

<210> 2
<211> 57
<212> RNA
<213> Unknown

<220>
<223> allosteric ribozyme

<400> 2
gcgucucuga ugagcucgau accagccgaa aggcccuugg cagcuaucga aacucug 57

<210> 3
<211> 62
<212> RNA
<213> Unknown

<220>
<223> allosteric ribozyme

<400> 3
gcgucucuga ugagccuugg gaagaaacug uggcacuucg gugccagcac gucgaaacuc 60

ug 62

<210> 4
<211> 13
<212> RNA
<213> Unknown

<220>
<223> substrate for ribozyme

<220>
<221>  modified_base
<222>  (1)..(1)
<223>  fluoresceine labelled

<220>
<221>  modified_base
<222>  (13)..(13)
<223>  dabcyl labelled

<400>  4
cagagucaga ugu                                                          13

<210>  5
<211>  13
<212>  RNA
<213>  Unknown

<220>
<223>  substrate for ribozyme

<220>
<221>  modified_base
<222>  (13)..(13)
<223>  dabcyl labelled

<400>  5
cagagucaga ugu                                                          13

## Description

The present invention is related to allosteric ribozymes, polynucleotides comprising preferably a hammerhead ribozyme moiety and a target binding moiety, a complex and a composition comprising such polynucleotides, methods for determining the presence and/or concentration of an analyte, methods for the generation of allosteric (deoxy) ribozymes and methods for the generation of a L-nucleic acid binding to a target molecule in a distinct enantiomeric form.

Analytical tasks are getting more and more abundant which is, among others, also based on the increased need to monitor the environment. In connection therewith, particularly the analysis of biological samples is of growing importance both in research and routine analysis. High throughput systems have been devised for such purpose which have to comply with the need for both specificity and sensitivity. In any case, the analysis of samples comprises two aspects, one being the detection aspect and the other one being the read-out aspect. For high throughput systems several approaches exist. One of them are enzyme based systems. Using this kind of system, however, there are serious limitations insofar as the particular analyte has to be somehow linked to a modification of the enzymatic activity. Only under very particular conditions the analyte corresponds to an effector to enzymatic activity so that a mediator system usually has to be introduced. One example for such a mediator system is the use of antibodies, more particular of monoclonal antibodies which may be generated for quite a number of analytes. Subsequent to the specific binding of the antibodies to the analyte, a distinct readout is necessary which may be an enzyme based system. Such kind of systems combine specificity and sensitivity, however, are difficult to design and operate. More particularly, for high throughput systems a further limitation arises from the mere fact that several compounds have to be added such as the antibody, the substrate, the enzyme, the enzyme substrate and the analyte. In addition, the generation of an antibody specific for a distinct analyte is not possible in every case. Furthermore, the interaction between the antibody as detecting system and the enzyme as readout system has to be carefully adapted for each individual analyte.

A further approach for a highly sensitive and specific analytical system is described in international patent application WO 98/27104. There bioreactive allosteric polynucleotides are described, the function or configuration of which may be modified with a chemical effector.

International patent application WO 00/26226 discloses multidomain polynucleotide molecular sensors which are closely related to the bioreactive allosteric polynucleotides. These multi-domain polynucleotides which are responsive to signalling agents, are designed and constructed to have at least three domains which can be partially or completely overlapping or non-overlapping, namely an actuator (catalytic or reporter) domain, a bridging domain, and a receptor domain. In some particular embodiments the catalytic domain is a ribozyme which is linked to a receptor domain which changes the activity of the ribozyme upon binding of its ligand.

These constructs, however, are composed of RNA and thus face degradation in biological samples. Because of this degradation a re-use of this kind of system is very difficult and a proper analysis, particularly if the reaction time extends over a significant period of time, may be affected due to an inactivation of the analytical device, i. e. the RNA constructs, which requires the extensive incorporation of negative and positive controls into such an assay format.

The problem underlying the present invention is thus to provide a highly specific and sensitive analytical device which allows the detection of any analyte, preferably in a biological sample thus overcoming the shortcomings of the analytical systems as described in the prior art, more particularly of the ribozyme based detection systems as disclosed in WO 00/26226.

According to a first aspect the problem is solved by an allosteric (deoxy) ribozyme, preferably a hammerhead (deoxy) ribozyme, characterized in that the ribozyme consists of L-nucleotides.

According to a second aspect the problem is solved by a polynucleotide comprising a (deoxy) ribozyme moiety, preferably a hammerhead ribozyme moiety, which comprises a catalytic domain and a binding site for a ribozyme substrate, and an target binding moiety, whereby the target binding moiety is specific for a target molecule, wherein the catalytic activity of the catalytic domain is reduced in the absence of the target molecule compared to the activity of the catalytic domain in the presence of the target molecule, characterized in that the polynucleotide consists of L-nucleotides.

According to a third aspect the problem is solved by a polynucleotide comprising a (deoxy) ribozyme moiety, preferably a hammerhead ribozyme moiety, which comprises a catalytic domain and a binding site for a ribozyme substrate, and a target binding moiety, whereby the target binding moiety is specific for a target molecule, preferably a polynucleotide according to the second aspect of the present invention, further comprising the target molecule bound to the target binding moiety, whereby the catalytic activity of the catalytic domain is increased in the presence of the target molecule compared to the activity of the catalytic domain in the absence of the target molecule, characterized in that the polynucleotide consists of L-nucleotides.

According to a fourth aspect the problem is solved by a polynucleotide comprising a (deoxy) ribozyme moiety, preferably a hammerhead ribozyme moiety, which comprises a catalytic domain and a binding site for a ribozyme substrate, and an target binding moiety, whereby the target binding moiety is specific for a target molecule, wherein the catalytic activity of the catalytic domain is increased in the absence of the target molecule compared to the activity of the catalytic domain in the presence of the target molecule, characterized in that the polynucleotide consists of L-nucleotides.

According to a fifth aspect the problem is solved by a polynucleotide comprising a (deoxy) ribozyme moiety, preferably a hammerhead ribozyme moiety, which comprises a catalytic domain and a binding site for a ribozyme substrate, and a target binding moiety, whereby the target binding moiety is specific for a target molecule, preferably a polynucleotide according to the fourth aspect of the present invention, further comprising the target molecule bound to the target binding moiety, whereby the catalytic activity of the catalytic domain is decreased in the presence of the target molecule of the aptamer compared to the activity of the catalytic domain in the absence of the target molecule, characterized in that the polynucleotide consists of L-nucleotides.

According to a sixth aspect the problem is solved by a polynucleotide comprising a (deoxy) ribozyme moiety, preferably a hammerhead ribozyme moiety, which comprises a catalytic domain and a binding site for a ribozyme substrate, and a target binding moiety, whereby the target binding moiety is specific for a target, more particularly a polynucleotide according to any of the aspects of the present invention, wherein the base pairing pattern of at least part of the polynucleotide in the presence of and/or upon binding of the target molecule is different from the base pairing pattern of the polynucleotide in the absence of and/or non-binding of the target molecule, characterized in that the polynucleotide consists of L-nucleotides.

In a preferred embodiment of any aspect of the present invention the polynucleotide further comprises a ribozyme substrate.

In another preferred embodiment of any aspect of the present invention the ribozyme substrate is a FRET-substrate.

In a further preferred embodiment of any aspect of the present invention the complex of ribozyme moiety and ribozyme substrate forms a quenching system.

In a more preferred embodiment the quenching system is formed by a fluorophor group and a quenching group.

In an embodiment of any aspect of the present invention the polynucleotide consists of L-RNA, L-DNA or mixtures thereof.

According to a seventh aspect the problem is solved by a complex comprising the polynucleotide and ribozyme according to any aspect of the present invention and a ribozyme substrate, preferably a ribozyme substrate for the ribozyme moiety of the polynucleotide.

In a preferred embodiment the complex further a target molecule, preferably a target molecule for the target binding moiety of the polynucleotide.

According to an eighth aspect the problem is solved by a composition comprising the polynucleotide and ribozyme, respectively, according to any of the aspects of the present invention and a ribozyme substrate, preferably a ribozyme substrate for the ribozyme moiety of the polynucleotide.

In a preferred embodiment the composition further comprises a target molecule, preferably a target molecule for the target binding moiety of the polynucleotide.

According to a ninth aspect the problem is solved by a biosensor comprising a polynucleotide and a ribozyme, respectively, according to any of the aspects of the present invention.

In a preferred embodiment the polynucleotide is immobilized on a support.

According to a tenth aspect the problem is solved by a method for determining the presence and/or concentration of an analyte comprising the steps of
a) providing an oligonucleotide and a ribozyme, respectively, according to any aspect of the present invention,
b) optionally determining the catalytic activity of the ribozyme moiety,
c) providing a substrate for the ribozyme moiety of the polynucleotide and reacting such substrate with the polynucleotide,
d) optionally determining the catalytic activity of the ribozyme,
e) adding a sample presumably containing the analyte,
f) determining whether the substrate is cleaved by the ribozyme moiety,
wherein the analyte is the target molecule of the target binding moiety of the polynucleotide.

According to an eleventh aspect the problem is solved by a method for determining the presence and/or concentration of an analyte comprising the steps of
a) providing an oligonucleotide and a ribozyme, respectively, according to any aspect of the present invention, whereby the ribozyme preferably comprises a substrate,
b) optionally determining the catalytic activity of the ribozyme moiety,
c) adding a sample presumably containing the analyte,
d) determining whether the substrate is cleaved by the ribozyme moiety,
wherein the analyte is the target molecule of the target binding moiety of the polynucleotide.

In a preferred embodiment of the tenth and eleventh aspect of the present invention the substrate comprises a fluorescent group and a quenching group and whereby after cleavage of the substrate by the catalytic domain of the ribozyme the quenching of the fluorescence is reduced.

According to a twelfth aspect the problem is solved by a kit comprising
a) an allosteric (deoxy) ribozyme according to the first aspect of the present invention and/or a polynucleotide according to any of the aspects of the present invention and, optionally,
b) a substrate for the ribozyme moiety of the polynucleotide and ribozyme, respectively, according to any of the aspects of the present invention.

According to a thirteenth aspect the problem is solved by a method for the generation of an allosteric L-(deoxy) ribozyme, preferably according to the first aspect of the present invention and/or a polynucleotide according to any aspect of the present invention, with an allosteric effector and/or a target molecule being a distinct enantiomer, comprising the following steps:
a) providing a D-polynucleotide, preferably a library of D-polynucleotides, whereby the polynucleotide comprises a (deoxy) ribozyme moiety, preferably a hammerhead ribozyme moiety, which comprises a catalytic domain, a binding site for a ribozyme substrate and a ribozyme substrate, and a candidate target binding moiety, whereby the candidate target binding moiety is of random sequence;
b) selecting for D-polynucleotide(s) which is/are not catalytically active in the absence of the optical antipode of the allosteric effector and/or of the target molecule;
c) contacting the selected D-polynucleotide(s) from step b) with the optical antipode of the allosteric effector and/or of the target molecule;
d) selecting the D-polynucleotide(s) the catalytic domain's activity of which is increased upon contacting and/or binding of the optical antipode of the allosteric effector and/or the target molecule; and
e) preparing L-polynucleotide(s) having a sequence identical to those D-polynucleotide(s) selected in step d).

According to a fourteenth aspect the problem is solved by a method for the generation of an allosteric L-(deoxy) ribozyme, preferably according to the first aspect of the present invention and/or a polynucleotide according to any of the aspects of the present invention, with an allosteric effector and/or a target molecule being a distinct enantiomer, comprising the following steps:
a) providing a D-polynucleotide, preferably a library of D-polynucleotides, whereby the polynucleotide comprises a (deoxy) ribozyme moiety, preferably a hammerhead ribozyme moiety, which comprises a catalytic domain, a binding site for a ribozyme substrate and a ribozyme substrate, and a candidate target binding moiety, whereby the candidate target binding moiety is of random sequence;
b) selecting for D-polynucleotide(s) which is/are catalytically active in the absence of the optical antipode of the allosteric effector and/or of the target molecule;
c) contacting the selected D-polynucleotide(s) from step b) with the optical antipode of the allosteric effector and/or of the target molecule;
d) selecting the D-polynucleotide(s) the catalytic domain's activity of which is decreased upon contacting and/or binding of the optical antipode of the allosteric effector and/or the target molecule; and
e) preparing L-polynucleotide(s) having a sequence identical to those D-polynucleotide(s) selected in step d).

According to a fifteenth aspect the problem is solved by a method for the generation of an allosteric L-(deoxy) ribozyme, preferably according to the first aspect of the present invention and/or a polynucleotide according to any of the aspects of the present invention, with an allosteric effector and/or a target molecule being a distinct enantiomer, comprising the following steps:
a) providing a L-polynucleotide, preferably a library of L-polynucleotides, whereby the polynucleotide comprises a (deoxy) ribozyme moiety, preferably a hammerhead (deoxy) ribozyme moiety, which comprises a catalytic domain, a binding site for a ribozyme substrate and a ribozyme substrate, and a candidate target binding moiety, whereby the candidate target binding moiety is of random sequence;
b) selecting for L-polynucleotide(s) which is/are not catalytically active in the absence of the allosteric effector and/or of the target molecule;
c) contacting the selected L-polynucleotide(s) from step b) with the allosteric effector and/or of the target molecule;
d) selecting the L-polynucleotide(s) the catalytic domain's activity of which is increased upon contacting and/or binding of the allosteric effector and/or the target molecule; and
e) preparing L-polynucleotide(s) having a sequence identical to those D-polynucleotide(s) selected in step d).

According to a sixteenth aspect the problem is solved by a method for the generation of an allosteric L-(deoxy) ribozyme, preferably according to the first aspect of the present invention and/or a polynucleotide according to any of aspects of the present invention, with an allosteric effector and/or a target molecule being a distinct enantiomer, comprising the following steps:
a) providing a L-polynucleotide, preferably a library of L-polynucleotides, whereby the polynucleotide comprises a (deoxy) ribozyme moiety, preferably a hammerhead (deoxy) ribozyme moiety, which comprises a catalytic domain, a binding site for a ribozyme substrate and a ribozyme substrate, and a candidate target binding moiety, whereby the candidate target binding moiety is of random sequence;
b) selecting for L-polynucleotide(s) which is/are catalytically active in the absence of the allosteric effector and/or of the target molecule;
c) contacting the selected L-polynucleotide(s) from step b) with the allosteric effector and/or of the target molecule;
d) selecting the L-polynucleotide(s) the catalytic domain's activity of which is decreased upon contacting and/or binding of the allosteric effector and/or the target molecule; and
e) preparing L-polynucleotide(s) having a sequence identical to those D-polynucleotide(s) selected in step d).

In a preferred embodiment of the thirteenth and fourteenth aspect the D-polynucleotids(s) is/are immobilized.

In a preferred embodiment of the fifteenth and sixteenth aspect the L-polynucleotids(s) is/are immobilized.

In a preferred embodiment of any of the thirteenth to sixteenth aspect of the present invention the random sequence has a length of about 20 to 80 nucleotides, preferably 30 to 60 nucleotides and more preferably 40 nucleotides.

In a seventeenth aspect the problem is solved by a method for the generation of a L-nucleic acid binding to a target molecule in a distinct enantiomeric form comprising
a) the steps a) to d) of the method according to any of aspects thirteen and fourteen of the present invention and embodiments thereof;
b) determining the target binding moiety of the polynucleotide(s) according step d) of the methods according to any of aspects thirteen and fourteen of the present invention and embodiments thereof; and
c) preparing L-polynucleotide(s) having a sequence identical to the target binding moiety of the polynucleotide(s) determined in step b).
wherein the target molecule in the distinct enantiomeric form corresponds to the allosteric effector and/or target molecule being a distinct enantiomer.

In an eighteenth aspect the problem is solved by a method for the generation of a L-nucleic acid binding to a target molecule in a distinct enantiomeric form comprising the steps
a) of the method according to any of claims aspects thirteen and fourteen of the present invention and embodiments thereof;
b) determining the target binding moiety of the polynucleotide(s) according step e) of the methods according to any of aspects thirteen and fourteen of the present invention and embodiments thereof;
c) preparing L-polynucleotide(s) having a sequence identical to the target binding moiety of the polynucleotide(s) determined in step b).
wherein the target molecule in the distinct enantiomeric form corresponds to the allosteric effector and/or target molecule being a distinct enantiomer.

In a nineteenth aspect the problem is solved by a method for the generation of a L-nucleic acid binding to a target molecule in a distinct enantiomeric form comprising the steps
a) of the method according to any of aspect fifteenth or sixteenth of the present invention and embodiments thereof;
b) determining the target binding moiety of the polynucleotide(s) according step d) of the methods according to any of aspect fifteenth or sixteenth of the present invention and embodiments thereof;
c) preparing L-polynucleotide(s) having a sequence identical to the target binding moiety of the polynucleotide(s) determined in step b).
wherein the target molecule in the distinct enantiomeric form corresponds to the allosteric effector and/or target molecule being a distinct enantiomer.

In a preferred embodiment of any of the thirteenth to nineteenth aspect of the present invention the target molecule in the distinct enantiomeric form and/or the allosteric effector in the distinct enantiomeric form is the naturally occurring form of the target molecule and/or of the allosteric effector.

The invention is based on the surprising finding that it is possible to generate allosteric ribozymes consisting of L-nucleotides, also referred to herein as allosteric L-ribozymes. A further description of this type of ribozymes is given in connection with the polynucleotides according to the present invention. In so far, any embodiment or use disclosed herein in relation to the inventive allosteric ribozymes applies also to the inventive polynucleotides and vice versa. This kind of allosteric ribozymes are also referred herein as allosteric spiegelzymes. Because of these spiegelzymes it is possible for the very first time to have a biologically stable analytical system which can easily be adapted to the individual analyte and has immediately attached thereto the read-out system which in the present case is the ribozyme moiety and in particular the catalytic activity in connection with a signal generating event or reaction. Also surprisingly a highly specific and sensitive applicable analytical tool which is factually stable in any biological system and applicable in a high throughput system, is thus provided.

This stability arises from the fact that naturally occurring nucleic acids and thus also the allosteric D-ribozymes as described in the prior art, are typically degraded by enzymes present in many biological samples. The use of L-nucleotides to construct the inventive ribozymes makes sure that this kind of ribozyme is not degraded by any nucleases which are specific to nucleotides comprised of D-nucleotides.

This stability of the allosteric L- ribozyme in a sample clearly renders much easier the analysis of chemical reactions or the determination whether a particular analyte is present in a sample.

As used herein ribozyme or ribozyme moiety, generally referred to herein as ribozyme, means any catalytically active nucleic acid. The catalytic activity may also be directed to the ribozyme itself such as in splicing. The ribozyme may be constituted of ribonucleotides and/or of deoxribonucleotides. The term (deoxy) ribozyme denotes any such ribozyme which is either comprised of ribuncleotides or deoxyribonucleotides or both of them. As long as not mentiones otherwise the term ribozyme as used herein shall be understood as (deoxy) ribozyme. If the ribozyme contains both ribonucleotides and deoxyribonucleotides each of the two species is preferably present as a sequence of severals of them. Such stretches may correspond to individual domains, either functional or sequential, of the ribozyme. However, it is also within the present invention that ribonucleotides and deoxyribonucleotides are arranged in any order or grouping within the ribozyme.

What has been said before in relation to the formation of the ribozyme out of ribonucleotides and/or deoxyribonucleotides applies also to the ribozyme substrate.

Any of the (deoxy) ribozymes or polynucleotides, including the ribozyme substrate, may be modified. Such modification may be related to the single nucleotide of the nucleic acid and are well known in the art. Examples for such modification may be taken from Kusser, W.(2000) J Biotechnol, 74: 27-38; Aurup, H. et al. **(1994)** *Nucleic Acids Res,* **22,** 20-4; Cummins, L.L. et al, **(1995)** *Nucleic Acids Res,* **23,** 2019-24; Eaton, B.E. et al. **(1995)** *Chem Biol,* **2,** 633-8; Green, L.S. et al., **(1995)** *Chem Biol,* **2,** 683-95; Kawasaki, A.M. et al., **(1993)** *J Med Chem,* **36,** 831-41; Lesnik, E.A. et al., **(1993)** *Biochemistry,* **32,** 7832-8; Miller, L.E. et al., **(1993)** *JPhysiol,* **469,** 213-43.

The ribozyme may actually be of any type of ribozyme such as hammerhead ribozymes, hammerhead-like ribozymes and hairpin ribozymes such as described, among others in Ruffner DE, Dahm SC, Uhlenbeck OC. Studies on the hammerhead RNA self-cleaving domain. Gene. 1989 Oct 15;82(1):31-41;Feldstein PA, Buzayan JM, van Tol H, deBear J, Gough GR, Gilham PT, Bruening G. Specific association between an endoribonucleolytic sequence from a satellite RNA and a substrate analogue containing a 2'-5' phosphodiester. Proc Natl Acad Sci U S A. 1990 Apr;87(7):2623-7; Hampel A, Tritz R, Hicks M, Cruz P. 'Hairpin' catalytic RNA model: evidence for helices and sequence requirement for substrate RNA. Nucleic Acids Res. 1990 Jan 25;18(2):299-304; and Tang J, et al. Proc Natl Acad Sci USA 2000 May 23; 97 (11): 5784-9. Hammerhead ribozymes and hammerhead-like ribozymes are, among others, described in Vaish, NK et al,; Proc Natl Acad Sci USA 1998 Mar 3; 95(5): 2158-62; Kore AR et al.; Nucleic Acids Res. 1998 Sep 15; 26 (18): 4116-20; Ludwig, J et al; Nucleic Acids Res 1998 May 15,26 (10): 2279-85; Kore AR et al. J Mol Biol 2000 Sep 1; 301 (5): 1113-21. Tang J, et al. Proc Natl Acad Sci USA 2000 May 23; 97 (11): 5784-9 describe various classes of ribozymes having, among others, phosphoesterase activity. As used herein, ribozymes also comprise deoxy ribozymes which are, among others, described in Breaker RR, Joyce GF. A DNA enzyme with Mg(2+)-dependent RNA phosphoesterase activity. Chem Biol. 1995 Oct;2(10):655-60; Breaker RR, Joyce GF. A DNA enzyme that cleaves RNA. Chem Biol. 1994 Dec;1(4):223-9; Santoro SW, Joyce GF, Sakthivel K, Gramatikova S, Barbas CF 3rd. RNA cleavage by a DNA enzyme with extended chemical functionality. J Am Chem Soc. 2000 Mar 22;122(11):2433-9; and Santoro SW, Joyce GF. A general purpose RNA-cleaving DNA enzyme. Proc Natl Acad Sci U S A. 1997 Apr 29;94(9):4262-6. Tang et al (supra) have selected a variety of different types of ribozymes. Other types of self-cleaving ribonucleic acids are, e.g. Neurospora VS RNA (Guo HC, De Abreu DM, Tillier ER, Saville BJ, Olive JE, Collins RA. Nucleotide sequence requirements for self-cleavage of Neurospora VS RNA. J Mol Biol. 1993 Jul 20;232(2):351-61); and RNA from human hepatitis delta virus (Kuo MY, Sharmeen L, Dinter-Gottlieb G, Taylor J. Characterization of self-cleaving RNA sequences on the genome and antigenome of human hepatitis delta virus. J Virol. 1988 Dec;62(12):4439-44.

The allosteric ribozymes of the present invention may have a catalytic activity which is either a phophoesterase activity or an activity such as a peptidyl-transferase activity (Zhang B, Cech TR. Peptidyl-transferase ribozymes: trans reactions, structural characterization and ribosomal RNA-like features. Chem Biol. 1998 Oct;5(10):539-53.), ester transferase activity (Jenne A, Famulok M. A novel ribozyme with ester transferase activity. Chem Biol. 1998 Jan;5(1):23-34.), amide synthase activity ( Wiegand TW, Janssen RC, Eaton BE. Selection of RNA amide synthases. Chem Biol. 1997 Sep;4(9):675-83.), carbon-carbon bond formation activity such as a Diels-Alderase activity (Tarasow TM, Tarasow SL, Eaton BE. RNA-catalysed carbon-carbon bond formation.Nature. 1997 Sep 4;389(6646):54-7.), an amino acid transferase activity (Lohse PA, Szostak JW. Ribozyme-catalysed amino-acid transfer reactions. Nature. 1996 May 30;381(6581):442-4.), an amidase activity (Dai X, De Mesmaeker A, Joyce GF.

Cleavage of an amide bond by a ribozyme. Science. 1995 Jan 13;267(5195):237-40.), a catalytic activity for carrying out the Michael reaction (Sengle G, Eisenfuhr A, Arora PS, Nowick JS, Famulok M. Novel RNA catalysts for the Michael reaction. Chem Biol. 2001 May;8(5):459-73.). Further catalytic activities shown by the ribozyme moiety of the allosteric ribozymes of the present invention is cleavage of carboxylic amides and esters. Another catalytic activity which the allosteric ribozymes and polynucleotides according to the present invention may exhibit is a ligase activiy which as such is described in Robertson MP, Ellington AD. In vitro selection of nucleoprotein enzymes. Nat Biotechnol. 2001 Jul;19(7):650-5; and Robertson MP, Ellington AD. Design and optimization of effector-activated ribozyme ligases. Nucleic Acids Res. 2000 Apr 15;28(8):1751-9.

Basically, the allosteric L-ribozyme according to the present invention and the polynucleotides according to the present invention have a similar design. More particularly, the inventive polynucleotide typically comprises a ribozyme moiety which comprises a catalytic domain and a binding site for a ribozyme substrate, and an target binding moiety, whereby the target binding moiety is specific for a distinct target molecule and the polynucleotide consists of L-nucleotides. In preferred embodiments the ribozyme and ribozyme moiety, respectively, is a hammerhead ribozyme. Alternatively, the ribozyme and ribozyme moiety, respectively, is a hairpin ribozyme. The two moieties, i.e. the ribozyme moiety and the target binding moiety may be clearly separated, overlapping, partially overlapping or linked to each other via a nucleotide sequence which is herein also referred to as linker moiety or bridging moiety. Due to this modular design, the inventive polynucleotide may exert the function as allosteric ribozyme.

It is to be acknowledged by the ones skilled in the art that the following illustration of the design of the ribozyme moiety particularly refers to a hammerhead ribozyme, hammerhead-like ribozymes and other ribozymes having a phosphodiesterase activity. However, this teaching applies equally to other types of ribozymes which are known in the art such as hairpin ribozymes and, at least partially, described above.

The ribozyme moiety is comprised of a catalytic domain and a binding site for a ribozyme substrate. This ribozyme substrate is typically a nucleic acid which is cleaved by the catalytic domain if the ribozyme or the catalytic domain is in an active state. This kind of active state is generated only upon binding or interaction of the allosteric effector or at least more prominent upon binding or interaction, generally referred to herein as binding, of the allosteric effector which means that the catalytic activity of the ribozyme moiety is increased. It is also within the present invention that the allosteric effector is actually an allosteric inhibitor to the catalytic activity of the ribozyme. Insofar the polynucleotide according to the present invention may be such as to comprise a ribozyme moiety, preferably a hammerhead ribozyme moiety, which comprises a catalytic domain and a binding site for a ribozyme substrate, and a target binding moiety, whereby the target binding moiety is specific for a target molecule, wherein the catalytic activity of the catalytic domain is increased in the absence of the target molecule compared to the activity of the catalytic domain in the presence of the target molecule whereby the polynucleotide consists of L-nucleotides. Accordingly, in the presence of the target molecule the catalytic activity of the domain is reduced compared to the activity of the catalytic domain in the absence of the target molecule. This allosteric effector is the target molecule against which the target binding moiety of the inventive polynucleotide is actually directed. Upon binding of the target molecule to the target binding moiety a conformational change in the target binding moiety occurs and, optionally also in other parts of the polynucleotide, such as the results in an increase of activity of the catalytic domain towards the ribozyme substrate. It is within the present invention that the catalytically inactive domain or ribozyme still shows some cleavage reaction towards the substrate or that the ribozyme moiety is catalytically active despite the absence of the target molecule, although the level of this catalytical activity is lower than the one in the presence of the allosteric effector, i.e. the target molecule of the target binding moiety. However, the catalytic activity may be factually zero in the absence of the target molecule.

Both the ribozyme moiety and the target binding moiety are typically connected through covalent linkages, preferably through phosphodiester linkages between the nucleotides at the end of what is regarded as the ribozyme moiety and the end of what is regarded the target binding moiety. In addition, the ribozyme and polynucleotide may comprise a substrate for the catalytically active domain of the ribozyme which may be covalently linked to any of the domains forming the ribozyme and polynucleotide according to the present invention, respectively. Such covalent linkage of a substrate means that the catalytically active domain performs an intramolecular reaction which is typically a cleavage reaction. Without such covalent linkage of the ribozyme substrate the reaction will be an intermolecular reaction.

In preferred embodiment the inventive polynucleotides and allosteric ribozymes comprise a hammerhead ribozyme moiety which as such is known from the art and, for example, described in Vaish, NK et al,; Proc Natl Acad Sci USA 1998 Mar 3; 95(5): 2158-62; Kore AR et al.; Nucleic Acids Res. 1998 Sep 15; 26 (18): 4116-20; Ludwig, J et al; Nucleic Acids Res 1998 May 15,26 (10): 2279-85; Kore AR et al. J Mol Biol 2000 Sep 1; 301 (5): 1113-21; Ruffner DE, Dahm SC, Uhlenbeck OC. Studies on the hammerhead RNA self-cleaving domain. Gene. 1989 Oct 15;82(1):31-41. It is also known which kind of substrate may actually be used for the catalytic activity of the ribozyme which mostly results from the base pair requirements of the binding site of the ribozyme and ribozyme moiety, respectively. Hammerhead ribozymes typically comprise three helices of which two are formed by hybridising to the complementary sequences of their "target RNA" which is basically the substrate as referred to herein, and the third helix is formed by a double strand within the ribozyme. The catalytic domain of the riboyzme comprises nucleotides which are typically arranged between the double stranded structures. If it is referred to target RNAs herein, this means that these are RNA substrates which may be cleaved in either cis or trans by the hammerhead ribozyme.

The target binding moiety which confers to the hammerhead ribozyme the feature of being allosterically regulated, may be generated according to methods such as described in international patent application PCT/EP97/04726 the disclosure of which is herein incorporated by reference. Due to the fact that the target binding moiety consists of L-nucleotides, this is actually a so-called spiegelmer or spiegelmer moiety. Spiegelmers are functional nucleic acids which are produced as follows starting from combinatorial DNA libraries with the DNA oligonucleotides of these libraries having a central stretch of about 10 to about 100 randomized nucleotides which are flanked by two primer binding sites at the 5' and 3' termini. The generation of such kind of combinatorial libraries is, for example, described in Conrad, R.C., Giver, L., Tian, Y. and Ellington, A.D., 1996, Methods Enzymol., Vol 267, 336-367. This kind of chemically synthesized single stranded DNA library may be transferred to a double stranded library using polymerase chain reaction. This double stranded library may already be used for a selection. Typically, however, the strands are separated into single strands using standard techniques resulting in single stranded libraries which are then used for the in vitro selection method in case it is a DNA selection (Bock, L.C., Griffin, L.C., Latham, J.A., Vermaas, E.H. and Toole, J.J., 1992, Nature, Vol. 355, 564-566). However, it is also possible to immediately use the synthetic DNA library in in vitro selections. In addition, an RNA library may be generated from the double stranded DNA, at least if a T7 promotor has been introduced into the double stranded DNA. Using this method it is possible to establish a library of 10¹⁵ and more DNA and RNA molecules. Each of these molecules exhibits a different sequence and thus a different three-dimensional structure. Applying the in vitro selection method it is possible to isolate one or several DNA or RNA molecules after several cycles of selection and amplification, optionally also comprising mutation, which shows significant binding activity towards a given target. It is within the scope of the present invention to use libraries of modified polynucleotides as described above. The targets may be viruses, proteins, peptides, nucleic acids, small molecules such as metabolites, drugs and other metabolites or other chemical, biochemical or biological components such as described in Gold, L., Polisky, B., Uhlenbeck, O. and Yarus, 1995, Annu. Rev. Biochem. Vol. 64, 763-797 and Lorsch, J.R. and Szostak, J.W., 1996, Combinatorial Libraries, Synthesis, Screening and application potential, ed. Riccardo Cortese, Walter de Gruyter, Berlin.

The method envisages that target binding DNA or RNA molecules from the originally used library are isolated and amplified after the selection step using polymerase chain reaction. In case of RNA selections a reverse transcription is to be made before the amplifying step using the polymerase chain reaction. A library which is enriched after the first selection round, may then be used for a further selection round so that the enriched molecules from the first selection round have a chance to become again by selection and amplification the dominant species. In addition, the step of the polymerase chain reaction offers the possibility to introduce new mutations in the amplification step, for example by varying the salt concentration. After sufficient selection and amplification rounds the binding nucleic acid molecules are prevailing. Thus an enriched pool of nucleic acids has been established the members of which may be individualized by cloning and characterized by subsequent sequence analysis using standard techniques. The sequences obtained are then analysed regarding their binding activity towards the target used for the selection process. This method for the generation of aptamers is also referred to as the SELEX process and is, for example, described in EP 0 533 838 the disclosure of which is incorporated herein by reference. The best binding molecules may then be truncated to reduce the molecules to the essential binding domain.

Given this basic mechanism spiegelmers, i. e. functional nucleic acids characterized in that they are at least partially, preferably completely, made of non-naturally L-nucleotides and binding to the target against which the corresponding aptamers were selected, may be generated. The particular feature of this method resides in the generation of enantiomeric nucleic acid molecules, i. e. of L-nucleic acid molecules which bind to a native target which is a native form or configuration of the target. The above described in vitro selection method is used to generate nucleic acids or sequences binding the enantiomer of the target molecule, i. e. the non-naturally occurring form of a naturally occurring target. In case the target molecule is a protein such a non-naturally occurring enantiomer would be the D-protein. The binding molecules thus obtained (D-DNA, D-RNA and respective D-derivatives) are sequenced and identical sequences are synthesized then using L-nucleotide monomers and L-nucleotide monomer derivatives. The thus obtained mirror image, enantiomeric nucleic acids (L-DNA, L-RNA and respective L-derivatives) and the so-called spiegelmers show a mirror image of the tertiary structure for reasons of symmetry and thus a binding characteristic for the target in its naturally form or configuration.

It is also within the scope of the present invention that an achiral target is used for the selection process. In such case any of the selected target binding D-nucleic acids may then be changed into the corresponding L-nucleic acid which will also bind to the achiral target molecule.

Because of this mechanism the target binding moiety of the allosteric L-ribozyme according to the present invention is actually a spiegelmer moiety. In addition, this target binding moiety is responsible for the flexibility and adaptability of the inventive allosteric L-ribozyme and allosteric polynucleotide and the biosensors comprising the same, which resides in the fact that it is possible to generate aptamers as well as spiegelmers against virtually any target molecule. The target binding moiety is thus responsible for the selective detection of the target molecule, i. e. the analyte, and the ribozyme moiety for the read-out. As the read-out is preferably the same, the inventive biosensors may be adapted to the various analytes simply by attaching the target specific target binding moiety to the ribozyme moiety.

In addition to the ribozyme moiety and the target binding moiety the inventive allosteric ribozyme and allosteric polynucleotide, respectively, may further comprise a bridging domain. The bridging domain may be needed to transmit the changes occurring to the target binding moiety upon binding of the target molecule, to the catalytic moiety thus increasing and reducing the catalytic activity of the ribozyme moiety, respectively.

This kind of bridging sequences are known in the art and, e. g., described in WO 00/26226 the disclosure of which is herein incorporated by reference.

It is to be noted that this kind of bridging sequence may either be an universal bridging sequence or bridging domain such as, among others, described in WO 00/26 22 6, or it may be selected from an originally random sequence such as also described in WO 00/26 22 6.

Without wishing to be bound the inventors currently assume that the base pairing pattern of at least a part of the allosteric ribozyme and the polynucleotides according to the present invention, respectively, and more particularly the differences thereof in case a target molecule is binding to the target binding moiety or not, is responsible to transmit a signal related to the binding event of the target molecule to the target binding moiety to the ribozyme moiety thus modulating the catalytic activity thereof. Particular contributions seem to be made by the intersection between the ribozyme moiety and the target binding moiety, and/or the bridging domain, if any.

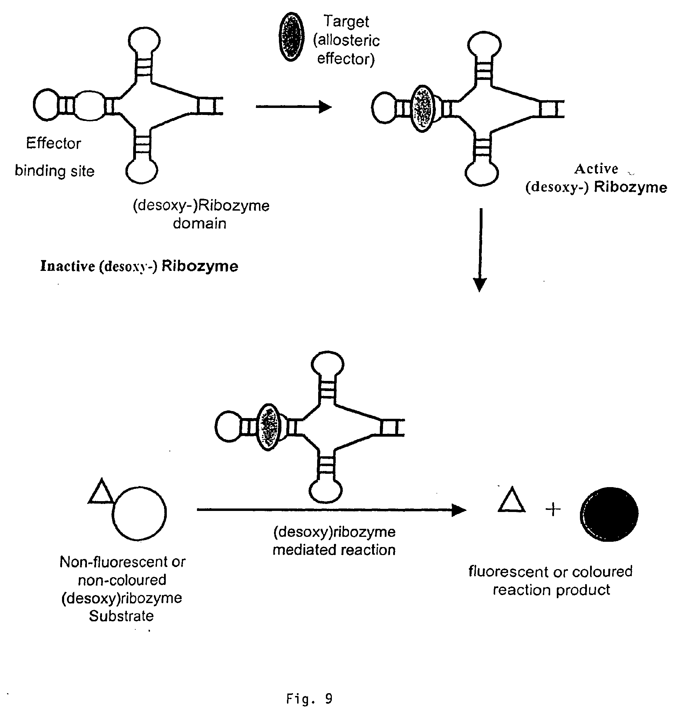

The ribozyme substrate is typically a nucleic acid sequence. The ribozyme substrate may, in principle, be independent from the other parts of the ribozyme and the polynucleotide according to the present invention be either L-DNA, L-RNA, mixtures thereof or derivatives thereof. This kind of substrate is used in case that the catalytic activity of the ribozyme is a hydrolysis of the phosphodiester linkage between two nucleotides forming the nucleic acid substrate. The substrate is preferably a so-called FRET substrate. However, it is also within the scope of the present invention that the catalytic activity of the ribozyme is related to a chemical reaction different from hydrolysis. For example ribozymes are known catalysing the Diels-Alder reaction. It is within the skills of the man of the art to provide substrates to monitor the catalytic activity of this kind of ribozyme. To monitor the catalytic activity of the allosteric ribozymes according to the present invention, a substrate may be used which, upon being subject to the particular catalytic activity of the ribozymes, is modified such as to generate a chromophore, luminophore, or fluorophore from a non-chromogenic, a non-luminophoric and non-fluorescent precursor substrate, respectively, as illustrated in Fig. 9.

FRET means Fluorescence Resonance Energy Transfer and refers to an energy transfer phenomenon in which the light emitted by the excited fluorescent group is absorbed at least partially by a fluorescence-modifying group. In a typical FRET experiment a nucleic acid is covalently labelled with two fluorophores, a donor and an acceptor, at different locations. The adsorption of the donor occurs at higher frequency than that of the acceptor. FRET involves a resonance between singlet-singlet electronic transition of the two fluorophores. This leads to a transfer of exitation energy from the donor to the acceptor. FRET can be observed in a variety of ways: including a reduction in the fluorescent quantum yield of the donor, a corresponding shortening of the donor exited state lifetime , and an increased fluorescent emission from the acceptor, (if fluorescent). If the fluorescence-modifying group, i.e. the acceptor, is a quenching group, then that group can either radiate the absorbed light as light of a different wavelength or it can dissipate it as heat. FRET depends on an overlap between the emission spectrum of the fluorescent group and the absorption spectrum of the quenching group. FRET also depends on the distance between the quenching group and the fluorescent group. Above a certain critical distance, the quenching group is unable to absorb the light emitted by the fluorescent group or can do so only purely. Typically, the substrate as used herein comprises an energy transfer pair. Preferred fluorescent groups are fluorescein, tetramethylrhodamin and 5-[(2-aminoethyl)amino]naphthalene-1-sulfonic acid (EDANS). The preferred quencher is (4-dimethylaminophenylazo)benzoic acid (DABCYL). However, as an alternative derivatives and analogs of DABCYL, i. e. of the dimethylaminophenylazobenzoic acids may be used. The same applies also the DABSYL and its derivatives and analogs which may replace DABCYL and its analogs and derivatives. When DABCYL and fluoresceine or EDANS are close enough together for FRET to occur, DABCYL absorbs light emitted from the fluoresceine or EDANS and dissipates the absorbed energy as heat. At separation greater than 60 Å, DABCYL is in many cases unable to quench the fluorescence from EDANS or fluoresceine. DABCYL itself is non-fluorescent at the wavelength used to excite EDANS or fluoresceine. Because of this the length of the substrate is limited to a certain extent or at least the distance between the fluorescent group and the quencher is limited. In any case, due to the cleavage by the catalytic domain of the ribozyme moiety of the inventive polynucleotide the fluorescent group and the quencher are separated resulting in emission of light depending on the characteristics of the fluorescent group. It is also within the present invention to some combination fluorescent dyes and quenchers such as a mixture of FRET and Fluorescence quenching, a system of a harvester fluorophore, an emitter fluorophore and a non-fluorescent quencher as described, for example in Tyagi S, Marras SA, Kramer FR. Wavelength-shifting molecular beacons. Nat Biotechnol. 2000 Nov;18(11):1191-6. Further technical teaching on how to realize FRET system may be taken from Tyagi S, Kramer FR. Molecular beacons: probes that fluoresce upon hybridization. Nat Biotechnol. 1996 Mar;14(3):303-8 or Tyagi S, Bratu DP, Kramer FR.

Multicolor molecular beacons for allele discrimination. Nat Biotechnol. 1998 Jan;16(1):49-53.

As possible fluorophores may be used cyanine dyes such as Cy3, Cy5, Cy5,5, and Cy7, or FAM (Carboxyfluoresceine), TET (Tetrachlorocarboxyfluoresceine), HEX (Hexachlorofluoresceine), TAMRA (Carboxytetramethylrhodamine), RHD (Carboxyrhodamine), ROX (Carboxy-X-rhodamine), JOE (6-carboxy-4',5'-dichloro- 2',7'-dimethoxyfluoresceine), EDANS, BODIPY, Lucifer yellow, Coumarin, TEXAS RED and Eosine. In addition, rare earth cryptate label may be used as fluorescence donors, such as, e.g., described in Lopez-Crapez E, Bazin H, Andre E, Noletti J, Greinier J, Mathis G. A homogeneous europium cryptate-based assay for the diagnosis of mutations by time-resolved fluorescence resonance energy transfer. Nucleic Acids Res. 2001 Jul 15; 29(14):E70. As an alternative to DABCYL, DABSYL may be used which is 4-dimethylaminoazobenzene-4'-sulfonyl may be used as quencher.

In addition to FRET also direct energy transfer is possible. Direct energy transfer refers to an energy transfer mechanism in which passage of a photon between the fluorescent group and the fluorescence-modifying group does not occur. Without being bound by a single mechanism, it is believed that in direct energy transfer, the fluorescent group and the fluorescence-modifying group interfere with each other's electronic structure. If the fluorescence-modifying group is a quenching group, this will result in the quenching group preventing the fluorescent group from even emitting light. Quenching groups and fluorescent groups are frequently close enough together if the ribozyme substrate is bound to the inventive oligonucleotide and allosteric ribozyme, respectively. Basically, the same groups as discussed in connection with FRET may be used for direct energy transfer. In addition, groups of fluorescent dyes and quencher dyes that do not even display FRET, such as Texas red and DABCYL, can be assumed to undergo direct energy transfer, leading to the efficient quenching of the fluorescent group by the other group. A detection system to monitor the catalytic activity of the ribozyme using this kind of quenching is also referred to as quenching system.

Regardless whether direct energy transfer or FRET is realized for generating a read-out signal, the groups required for this kind of energy transfer may be either both localized on the same molecule or on different molecules. In case both groups are arranged on the same molecule, they are preferably arranged on the substrate. It is within the present invention that at least one group is arranged at either the 5' end or the 3' end of the substrate. Preferably, one group is arranged at the 5' end and the other group is attached at the 3' end of the substrate. However, it is also within the scope of the present invention that one group is either arranged at the 3' end or the 5' end and the second group is arranged within the substrate, i.e. attached to a nucleotide different from the 3' and/or 5' terminal end of the substrate. In addition, it is also within the scope of the present invention that both groups are arranged within the substrate.

In a different embodiment at least one of the groups is attached to the allosteric L-ribozyme. Preferably, the second group for establishing a FRET or direct energy transfer system is attached to the substrate. This second group may be attached to either the 5 ' or 3' terminal nucleotide of the substrate or any non-terminal nucleotide of the substrate.

The inventive allosteric L- ribozyme and allosteric polynucleotides, respectively, may be present as a complex, either comprising a ribozyme substrate for the ribozyme moiety or a target molecule for the target binding moiety or a combination of both. Typically, the various constituents of such complex are linked to each other by non-covalent binding. Depending on the particular composition of such complex it may either be catalytically active or non-active. The same applies basically also to the inventive composition.

As has been outlined herein it is possible to generate the allosteric ribozyme and the polynucleotides according to the present invention, respectively, by rational design with the ribozyme moiety sequence and the substrate sequence as such being known from the art, only have to be synthesized using L-nucleotides rather than D-nucleotides. The target binding moiety which is actually a spiegelmer moiety, may be produced according to the technical teaching as described in the international patent application PCT/EP97/04726. The same applies also to the bridging sequence or bridging domain, respectively.

Alternatively, the allosteric ribozyme and the polynucleotides according to the present invention, respectively, may be produced by selection and more particularly by allosteric selection. These two techniques may also be applied to generate allosteric ribozymes and the polynucleotides according to the present invention, respectively, generated by rational design.

Basically, the selection method for allosteric ribozymes and the polynucleotides according to the present invention, respectively, which are regulated by an allosteric effector and a target molecule, respectively whereby the effector and the target molecule being a distinct enantiomer, comprises the following steps:
a) providing a D-polynucleotide, preferably a library of D-polynucleotides, whereby the polynucleotide comprises a (deoxy) ribozyme moiety, preferably a hammerhead ribozyme moiety, which comprises a catalytic domain, a binding site for a ribozyme substrate and a ribozyme substrate, and a candidate target binding moiety, whereby the candidate target binding moiety is of random sequence;
b) selecting for D-polynucleotide(s) which is/are not catalytically active in the absence of the optical antipode of the allosteric effector and/or of the target molecule;
c) contacting the selected D-polynucleotide(s) from step b) with the optical antipode of the allosteric effector and/or of the target molecule;
d) selecting the D-polynucleotide(s) of which the catalytic domain's activity is increased upon contacting and/or binding of the optical antipode of the allosteric effector and/or the target molecule; and
e) preparing L-polynucleotide(s) having a sequence identical to those D-polynucleotide(s) selected in step d).

As an alternative the selection method may comprise the following steps leading to an allosteric ribozymes and the polynucleotides according to the present invention, respectively, which are regulated by an allosteric effector and a target molecule, respectively whereby the ribozyme catalytic activity is reduced in the presence of the target molecule.
a) providing a D-polynucleotide, preferably a library of D-polynucleotides, whereby the polynucleotide comprises a (deoxy) ribozyme moiety, preferably a hammerhead ribozyme moiety, which comprises a catalytic domain, a binding site for a ribozyme substrate and a ribozyme substrate, and a candidate target binding moiety, whereby the candidate target binding moiety is of random sequence;
b) selecting for D-polynucleotide(s) which is/are catalytically active in the absence of the optical antipode of the allosteric effector and/or of the target molecule;
c) contacting the selected D-polynucleotide(s) from step b) with the optical antipode of the allosteric effector and/or of the target molecule;
d) selecting the D-polynucleotide(s) of which the catalytic domain's activity is decreased upon contacting and/or binding of the optical antipode of the allosteric effector and/or the target molecule; and
e) preparing L-polynucleotide(s) having a sequence identical to those D-polynucleotide(s) selected in step d).

It is to be noted that the allosteric effector, which as used herein comprises both the allosteric effector of the allosteric ribozyme and the target molecule of the target binding moiety of the polynucleotide according to the invention, is typically the one which occurs naturally and is referred to herein as the distinct enantiomer. This discrimination is made to render clear the fact that in the above described method not this form of the allosteric effector is used but the optical antipode thereof. If, for example, the allosteric effector shall be a L-protein, the above methods will use the corresponding D-protein as the optical antipode. In so far it may be referred to the prior art basically describing the method for the generation of aptazymes such as international patent application WO 00/26226 and European patent application EP 1 092 777 A1.

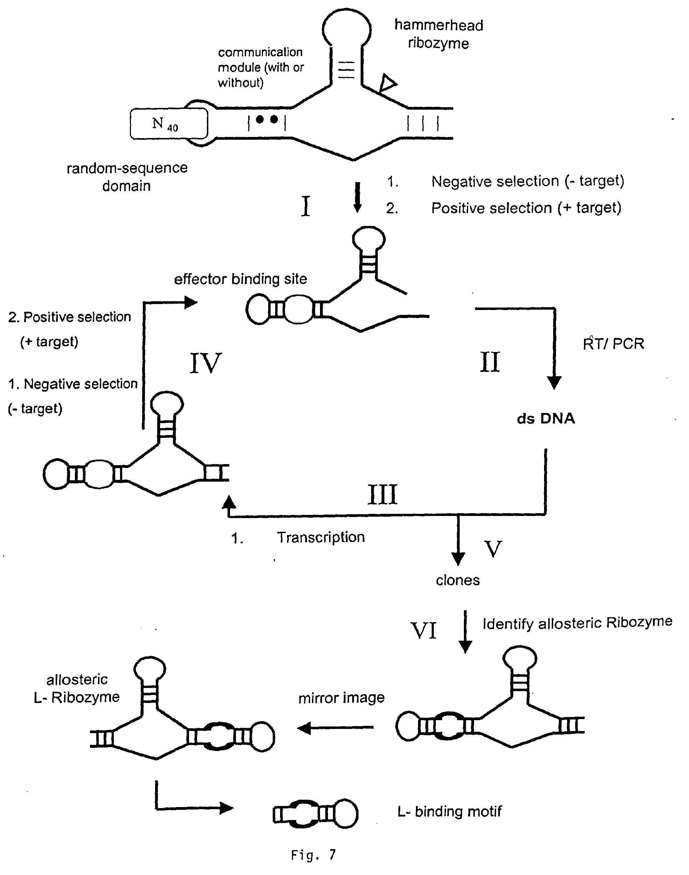

Besides the above mentioned steps further measures are helpful to carry out the selection of method which will be described in the following with reference to Fig. 7 for purpose of illustration only.

Basically, it is possible to carry out the method for selection of the allosteric ribozymes and polynucleotides according to the present invention starting from a library or a pool comprising a plurality of such polynucleotides. The use of such pool is clearly advantageous with regard to speed and efforts required for a successful screening. The members of the pool differ in the random sequence with the pool preferable having some 10¹² to 10¹⁸, preferably 10 ¹³ to 10¹⁶ and more preferably 10¹³ to 10¹⁵ differing in the random sequence. In the following the procedure is described starting from a pool of polynucleotides. Preferably the members of the library differ in the random sequence whereas the other part(s) of the polynucleotides are identical and more preferably have a catalytically active (deoxy) ribozyme (moiety) or domain.

In a first step a pool of polynucleotides having the size and with the features as described above is prepared of different double-stranded D-deoxyribonucleotide molecules. The random sequences may be prepared, for example, by using a nucleic acid synthesizer. In a second step of the above method, the D-DNA molecule pool is transcribed into a D-RNA molecule pool and thus the D-polynucleotide (library) provided corresponding to step a). Either the transcription or providing the D-RNA shall be done in the absence of the allosteric effector under conditions where only those D-RNA molecules shall be provided which are catalytically not active in the absence of the allosteric effector. This means that from the original pool of D-polynucleotides only those shall be subject to the selection which are not catalytically active in the absence of the allosteric effector.

In an embodiment of step b) the catalytically inactive RNAs are selected and isolated. Isolation may be done by PAGE (negative selection). These catalytically inactive RNAs are then exposed to or contacted with the optical antipode of the allosteric effector. Those RNAs being catalytically active in the presence of said antipode (positive selection) will then be further amplified by RT-PCR (Fig. 7 (II)) to generate double stranded DNA templates. The resulting DNAs are transcribed using bacteriophage T7 RNA polymerase (T7 RNAP) to generate a new population of RNA molecules that are (Fig. 7 (IV)) subject to the next round of negative and positive selections. Double-stranded DNAs from the desired rounds of selection are cloned and sequenced for further analysis. The T7 promotor sequence is introduced in every round of selection during PCR. T7 represents a double-stranded promotor sequence for T7 RNAP.

The catalytic activity of the ribozyme may be determined using, in principle, any suitable substrate. Particularly preferred is a substrate which is covalently linked to the ribozyme which allows for a rapid screening without additional steps of providing a substrate molecule although this embodiment of the selection method is also within the scope of protection of the present invention.

Preferably, the selection steps a) and b), respectively, to d) are repeated several times with the polynucleotide(s) selected in step d) being amplified and optionally varied again in their random sequence, and then being subject to another round of steps b) to d). After the last round of steps a) and b), respectively, to d), step e) is performed. Prior to step e) the sequence of the D-polynucleotides selected in step d) may be determined by any sequencing method known in the art. In a preferred embodiment this cycle is repeated up to 1 to 100 times.

The above selection method results in the generation of allosteric ribozymes of which the activity is increased in the presence of the allosteric effector. However, it is also within the scope of the present invention to provide allosteric ribozymes of which the activity is increased in the absence of the allosteric effector. For this kind of allosteric ribozyme the allosteric effector is actually an allosteric inhibitor. The above sequence of steps may be easily adapted for this kind of allosteric ribozyme. Accordingly, a step a) identical to step a) of the method for the selection of an allosteric ribozyme which shows an increased activity in the presence of an allosteric effector, is followed by a step b) where those D-polynucleotides which are catalytically active in the absence of the optical antipode of the allosteric effector and/or the target molecule, are selected. As step c) the D-polynucleotides selected in step b) are contacted with the optical antipode of the allosteric effector and/or the target molecule, respectively. As step d) those D-polynucleotides the catalytic domain's activity of which is increased upon contacting or binding of the optical antipode of the allosteric effector and/or the target molecule is selected. As step e) (an) L-polynucleotide(s) having a sequence identical to those D-polynucleotide(s) selected in step d) are prepared. Preferably such preparing is done by chemical synthesis.

The above disclosed embodiments and measures to be taken as described for the generation of allosteric ribozymes of which the activity is increased by an allosteric effector apply also to the generation of allosteric ribozymes the activity of which is decreased by an allosteric effector.

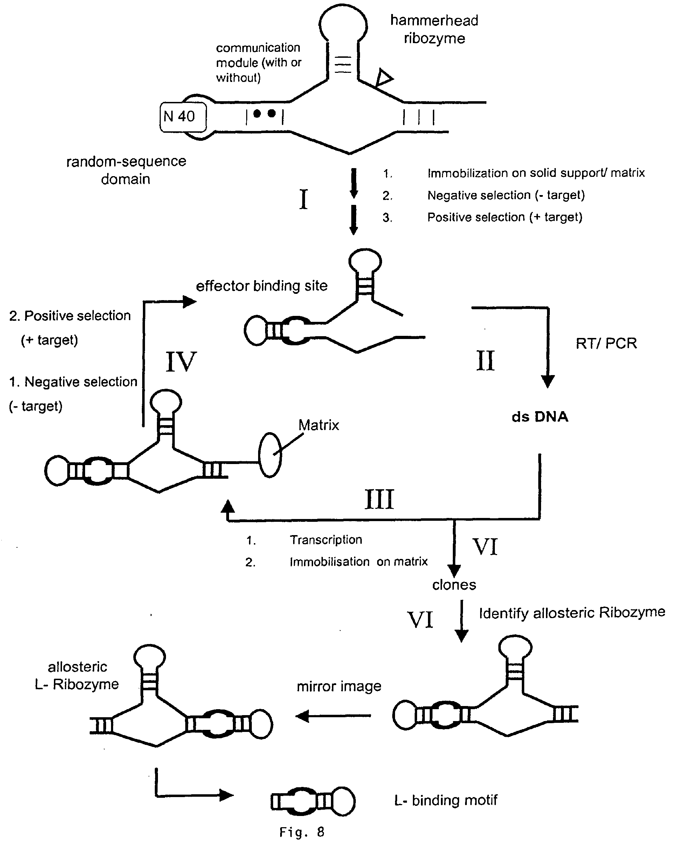

In a further embodiment of the above described methods for the generation of allosteric ribozymes by means of selection the D-polynucleotide and the polynucleotide pool is immobilized prior to any selection steps (negative as well as positive selection). It is also within the scope of the present invention that after any amplification or transcription the polynucleotide is also immobilized as depicted in Fig. 8. The advantage of this kind of immobilization resides in the fact that the otherwise necessary procedures such as gel electrophoresis become void which allows a faster selection and goes along with less losses. Such immobilisation may be either covalent or non-covalent. Non-covalent immobilisation may be done by using a biotin streptavidine or neutravidine or any other biotin binding moiety system, whereas covalent immobilisation may, e.g. be done by oxidation using periodate and a hydrazide modified solid support or by immobilization of a thiophosphate moiety of the respective nucleic acid with activated thiol solid support.

It is also within the scope of the present invention to select the allosteric ribozymes and polynucleotides, respectively, making use of a library of L-nucleotides instead of D-nucleotides. In such an approach the actual target rather than the optical antipode shall be used in the screening. As the screening and more particularly the subsequent steps of amplification would require an enzyme activity also acting on L-nucleotides which, however, is currently not available, a process of chemical amplification is used such as described in international patent application PCT/ DE 99/03856. In generally speaking, the following steps are taken: a) a first nucleic acid is immobilized on a first surface of a solid phase, b) a solution containing, preferably among others, a second nucleic acid binding to the first nucleic acid; c) transferring the second nucleic acid to an additional surface and immobilizing the second nucleic acid at that location; d) attaching a third nucleic acid which is again complementary to the second nucleic acid to the immobilized second nucleic acid; and e) transferring the third nucleic acid to a surface and immobilizing the same at that location.

The above described selection methods for the generation of allosteric ribozymes and the allosteric ribozymes and polynucleotides according to the present invention may also be used as the starting material for the generation of spiegelmers, i.e. L-nucleic acids or L-polynucleotides which are binding to a target molecule in a distinct enantiomeric form. As mentioned in connection with the inventive selection methods for the generation of allosteric ribozymes, these method make use in the selection process of the optical antipode of the target molecule against which the spiegelmer shall actually be directed, i.e. which shall actually be bound by the spiegelmer. Taken the modular design of the allosteric ribozymes as disclosed herein, the target binding moiety of the allosteric ribozyme and polynucleotides according to the present invention is actually a spiegelmer. Therefore, it is possible to determine or identify the target binding moiety either of the selected D-oligonucleotide of step d) of said methods and subsequently prepare the L-polynucleotide having a sequence identical to the target binding moiety, or to determine or identify the target binding moiety of the L-polynucleotide prepared in step e) of said methods and prepare such moiety. In both cases the target specific L-polynucleotide, i.e. the spiegelmer will be obtained.

The spiegelmers thus obtained may be further used or modified as known by the ones skilled in the art.

Either the allosteric L-ribozymes, the allosteric polynucleotides, the complex or the composition according to the present invention may be used as a biosensor. As discussed in connection with the characteristics of the inventive polynucleotides and allosteric L-ribozymes, respectively, the characteristic of the biosensor is its specificity, whereby the specificity is conferred to the biosensor by the specificity of the target binding moiety of the inventive polynucleotides. The biosensor may be present in solution or immobilized on a surface such as a support. Taken the particular advantage conferred by the inventive polynucleotides and allosteric L-ribozymes, respectively, an immobilized biosensor is preferred with regard to the extreme stability of the inventive polynucleotides which allows a more or less indefinite re-use of the immobilized biosensor without any loss of sensitivity and specificity

It is also within the present invention, however, to have several of the inventive allosteric L-ribozymes and allosteric polynucleotides in one batch such as a test tube, either in solution or immobilized on a surface, which preferably differ from each other in both the target binding moiety, the ribozyme moiety and preferably also in regard to the substrate specific for the ribozyme being usually specific to the individual ribozyme moiety. Because of this the presence of a distinct target will activate only the particular allosteric ribozyme having the respective target specific target binding moiety and thus providing for a target specific readout.

This kind of immobilized allosteric L-ribozymes and allosteric polynucleotides, respectively, immobilized on a surface are also referred herein as biochips. The immobilization may be done via standard nucleic acid immobilization techniques. Possible support material may be chosen from the group comprising glass, controlled pore glass, gold and plastics. This kind of support material may further be coated such as, e.g. by poly-Lysine, aminosilane (silanated), aldehyd-silane (silylated), epoxyactivated, Streptavidine, gold, polyacrylamide pads (aldehyde activated), agarose- aldehyde active.

The immobilization procedure may use a biotin streptavidine system or the like or covalent immobilization systems such as S-S linkages, each known to the one skilled in the art.

Using immobilized biosensors as disclosed herein the read out system may be fluorescence, FRET or radioactivity, such as ³²P, ³⁵S, ¹⁴C and ³H. In case of using radioactivity the read out could be done such as to measure the corresponding positions before and after the incubation with analyte as the part of the ribozyme substrate cleaved of by the catalytic activity of the ribozyme and ribozyme moiety, respectively, is carrying the label. The signal does generated is a decline in radioactivity. Preferably radioactivity as a read-out system is used in an intramolecular system, i.e. a system where the substrate is covalently linked to the allosteric ribozyme and ribozyme moiety, respectively.

Any of the inventive compounds such as the allosteric L-ribozyme, the allosteric polynucleotide, the complex comprising the same and the composition comprising the same as well as the biosensor may actually be used in a method for determining the presence and/or absence and/or concentration of an analyte. The specificity for the analyte is conferred to such an assay or analytical tool by the target specificity of the target binding moiety of the inventive polynucleotide. It is obvious for the one skilled in the art that the order of the steps to be realized as described in the inventive methods may vary depending on the particular circumstances and needs of the individual tests to be performed. Accordingly, positive as well as negative controls may be added and preferably in case of a kinetic analysis using the inventive biosensor the baseline is to be determined which may be done at any of the various steps. The resulting kinetic analysis of a sample may be compared to a standard curve for known concentrations of the analyte.

A sample presumably containing the analyte is preferably a biological sample such as blood, liquor, urine, sputum or any other body fluid. It is within the present invention that it is either known that the particular analyte is contained in the sample or it is not known whether the particular analyte is contained in said sample. The sample as such may be subject to a pretreatment which is known to the one skilled in the art. In any case and more particularly in connection with the inventive methods the step of determining whether the ribozyme substrate is cleaved by the ribozyme moiety can be performed in a manner making use of the fact that the catalytic domain of the ribozyme is active only in the of the analyte, i. e. the target molecule of the target binding moiety. However, it is also within the scope of the present invention that the ribozyme is only active in the absence of the target molecule and inactive in the presence of said target molecule. In the first system the target molecule acts as an allosteric activator whereas in the second system the target molecule acts as an allosteric inhibitor. One preferred system for determining this will be the use of a FRET substrate with the fluorescence being observable upon cleavage of the substrate by the catalytic domain of the ribozyme moiety and the presence of the target molecule of the target binding moiety. However, it will be appreciated by the ones skilled in the art that for this kind of method basically any and each suitable substrate or combination of different substrates or combination of labelled substrate and/or labelled ribozyme, particularly as disclosed herein, may be used. In a preferred embodiment of the present invention a combination of the biosensors is used. Typically, these biosensors differ from each other in the specificity of the target molecule conferred to them by the target binding moiety. In addition, the biosensors differ in the substrate specificity such that the binding of the target specific for a first biosensor results in reaction of the first biosensor with its specific substrate giving a distinct first signal, whereas binding of the target specific for a second biosensor results in reaction of the second biosensor with its specific substrate giving a distinct second signal. The distinct first and distinct second signal differ from each other allowing an unambiguous correlation with the presence or absence of the first and second target molecule. Basically, the number of different biosensors contained in such a combination of biosensors is only limited by the number of different signals which can be generated.

It is particularly preferred to have the above described combination of biosensors immobilized on a solid surface thus forming an array. Preferably at each distinct site of the surface or array only one type of biosenor, i.e. with one distinct target specificity, is immobilized. Under such conditions the signal generated upon the binding of the specific target molecule may be the same or different. If it is the same knowing that a specific biosensor is located at a specific site of the array allows to determine the presence or absence of a target molecule for any of the biosensors attached on the array.

The inventive methods are carried out under conditions which allow for the performing of the required reactions such as hybridisation of the substrate to the substrate binding site of the ribozyme moiety, binding of the target molecule to the target binding moiety and cleavage of the substrate. Respective reaction conditions are known to the one skilled in the art and, e. g., described in Dahm SC, Uhlenbeck OC., Role of divalent metal ions in the hammerhead RNA cleavage reaction, Biochemistry. 1991 Oct 1;30(39):9464-9; and Stage-Zimmermann TK, Uhlenbeck OC, Hammerhead ribozyme kinetics, RNA. 1998 Aug;4(8):875-89.

In a further aspect the invention is related to a kit which comprises either an allosteric L-ribozyme according to the invention or a polynucleotide according to the invention or complexes or compositions comprising the same. In addition, such kit may comprise a substrate for the ribozyme moiety of the polynucleotide and allosteric ribozyme according to the present invention. Furthermore, such kit may comprise buffers and other ingredients necessary to carry out the inventive methods. The polynucleotides may be present as solid or as a liquid solution thereof. The inventive kit is preferably used for the determination of the presence and/or absence and/or concentration of an analyte with the analyte preferably being identical to the target molecule of the target binding moiety of the allosteric ribozyme and allosteric polynucleotides, respectively, according to the present invention.

The details on how to perform such inventive method are illustrated in the examples and may be taken therefrom.

The invention is now further illustrated referring to the figures, the examples and the sequence listing from which further features, embodiments and advantages of the invention may be taken.
- Fig. 1: shows a theophylline biosensor according to the present invention comprising an inventive polynucleotide and a substrate, whereby the substrate is a FRET substrate.
- Fig. 2: shows the relative increase of fluorescence as a function of the theophylline concentration using the biosensor as described in Fig. 1.
- Fig. 3: shows a theophylline biosensor according to the present invention comprising an inventive polynucleotide and a substrate, whereby the 5' end of the polynucleotide is labelled with a fluoresceine group and the 3' end of the substrate is labelled with DABCYL.
- Fig. 4: shows the relative increase of fluorescence as a function of the concentration of theophylline using the biosensor according to Fig. 3.
- Fig. 5: shows an adenosine biosensor according to the present invention comprising an inventive polynucleotide and a substrate, whereby the substrate is a FRET substrate.
- Fig. 6: shows the relative increase of fluorescence as a function of the L-adenosine concentration using the biosensor as described in Fig. 5.
- Fig.7: shows the selection method for the generation of the allosteric ribozymes according to the present invention.
- Fig. 8: shows the selection method for the generation of the allosteric ribozymes according to the present invention using in immobilization step.
- Fig. 9: shows an illustration of the principle on which the use of the allosteric ribozymes according to the present invention as biosensors is based.
- Fig. 10: shows an illustration of the isolation of allosteric (deoxy) ribozymes.

An embodiment of the use of the allosteric ribozymes and allosteric ribozymes according to the present invention as biosensor is illustrated in Fig. 9. An inactive L-(desoxy)ribozyme becomes active only in the presence of a target molecule (analyte, allosteric effector). The active ribozyme catalyzes a reaction where a non-fluorescent or non-coloured substrate is modified to a fluorescent or coloured reaction product, which can be detected using standard techniques. This system is responding to the presence of the analyte (target molecule) and thus allows the determination of the concentrations of the analyte in a sample.

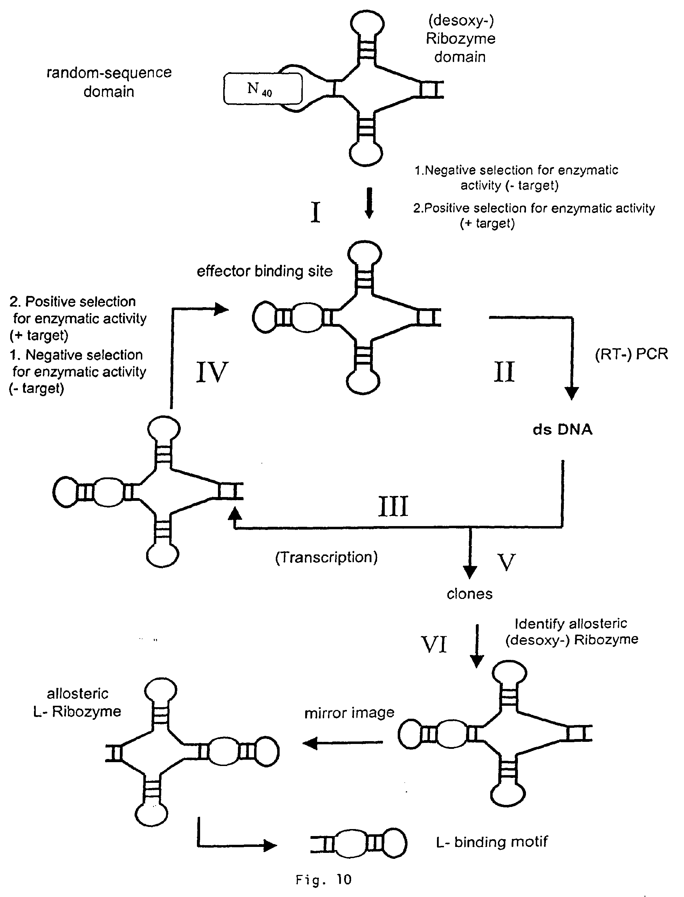

Fig. 10 illustrates the basic procedure for the isolation of any allosteric (deoxy) ribozymes. Thus, this selection procedure is not limited to hammerhead system but may be applied for any of the ribozyme species as described herein. In addition it is to be noted that using this kind of procedure any of the catalytic activities as described herein may be screened for, including but not limited to the ligase activity. In principle the basic steps are the ones as described in connection with fig. 7.

### Example 1: Preparation and sequences of various allosteric ribozymes and substrates thereto

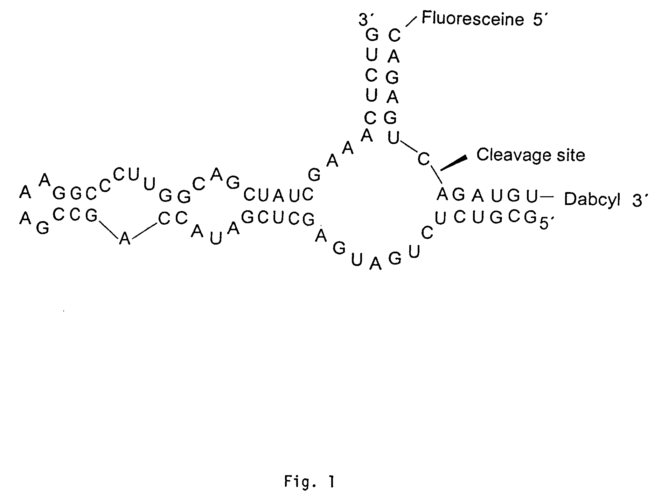

The individual L-oligonucleotides forming the biosensors as depicted in figures 1, 3 and 5 as well as the respective substrates were prepared on an ABI 394 DNA synthesizer (Applied Biosystems) at 0.2 µmol scale. L-RNA phosphoramidites were purchased from ChemGenes.

For SEQ ID NO: 4 and SEQ ID NO: 5 Molecular Beacon Icaa CPG (ChemGenes) was used. The fluoresceine modification was introduced during synthesis using the fluoresceine phosphoramidite (Glen Research).

All L-oligonucleotides were purified on denaturing PAGE as described in Sambrook et al. (Sambrook, Fritsch, Maniatis, Molecular Cloning- A laboratory Manual, 2^{nd} Ed. 1989, Cold Spring Harbor Laboratory Press.)

The following sequences were used, whereby SEQ ID NO: 1, SEQ ID NO: 2, SEQ ID NO: 3 and SEQ ID NO: 6 are all allosteric ribozymes as disclosed herein and SEQ.ID.No 4 and 5 are substrates to the ribozymes.

SEQ ID NO: 1
Fluoresceine- GCG UCU CUG AUG AGU CGU AUA CCA GCC GAA AGG CCC UUG
GCA GAU UCC GAA ACU CUG

SEQ ID NO: 2
GCG UCU CUG AUG AGC UCG AUA CCA GCC GAA AGG CCC UUG GCA GCU AUC
GAA ACU CUG

SEQ ID NO: 3
GCGUC UCU GAU GAG CCUU GGG AAG AAA CUG UGG CAC UUC GGU GCC AGC
A CGU CGA AAC UCU G

SEQ ID NO: 4
Fluoresceine- CAG AGU C AGA UGU -Dabcyl

SEQ ID NO: 5
CAG AGU C AGA UGU -Dabcyl

### Example 2: Allosteric ribozyme based biosensors for theophylline

This theophylline specific biosensor was constructed by combining the allosteric L-ribozyme comprising a nucleic acid sequence according to SEQ ID NO: 2 and a substrate comprising a nucleic acid sequence according to SEQ ID NO: 4. The allosteric L-ribozyme comprises a spiegelmer specific for theophylline as the target binding moiety corresponding to an aptamer moiety of an allosteric D-ribozyme. The complex of the allosteric L-ribozyme and the substrate is shown in Fig. 1.

The method of manufacture and performing a test for determining the impact of various concentrations of the target molecule, i.e. theophylline, on the observed fluorescence which could be used as a calibration curve in subsequent analysis for theophylline, can be summarized as follows:

600 pmol SEQ ID NO: 2 (or 600 pmol SEQ ID NO: 2 ) and 1000 pmol SEQ ID NO: 4 in 42 µl water were denatured for 3 min at 95°C and renatured for 5 min at 37°C.

6.5 µl of the obtained mixture were mixed with 10 µl buffer A (1500 mM NaCl, 40 mM KCI, 100 mM Hepes-Na pH 7.4), 40 µl 50 mM MgCl₂, 10 µl of a theophylline stock solution (concentration 1-50 mM) and 20 µl water. The Fluorescence measurements were done at a FLUOSTAR (BMG ). Samples were analysed in a black 96 well plate (Costar) at 490 nm Excitation and 525 nm Emission wave length (40 pulse per cycle, 100 cycles a 10 sec(or 50 cycles a 20 sec).

The time resolved Fluorescence was recorded and analysed. The original data are shown in table 1.

**Table 1:**

| nmol Theophylline per sample | relative increase of Fluorescence (ΔrFU) |
|---|---|
| 0 | 1132 |
| 10 | 3000 |
| 50 | 5245 |
| 100 | 6491 |
| 500 | 8019 |

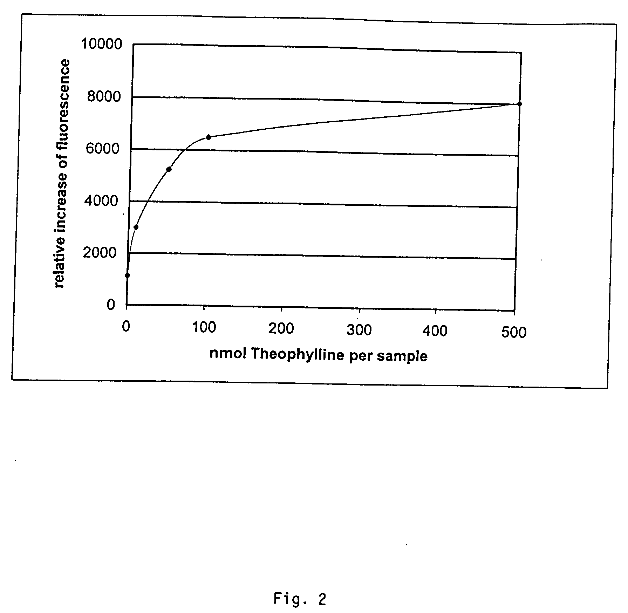

The results of this assay are also depicted in Fig. 2 .

### Example 3: Allosteric ribozyme based biosensors for theophylline

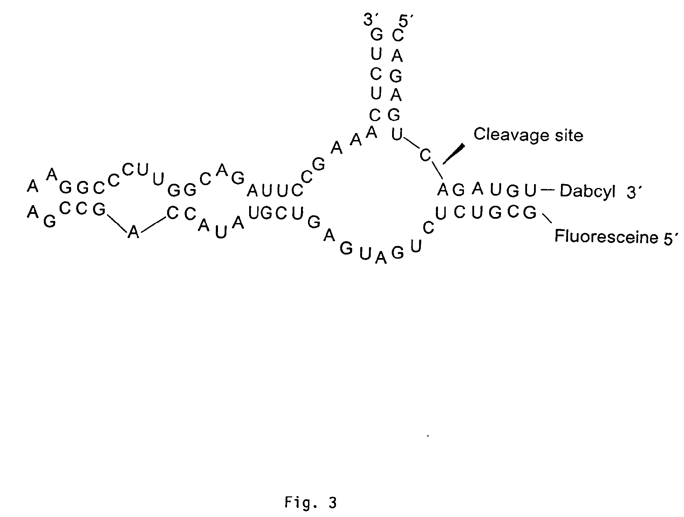

This theophylline specific biosensor was constructed by combining the allosteric L-ribozyme comprising a nucleic acid sequence according to SEQ ID NO: 1 and a substrate comprising a nucleic acid sequence according to SEQ ID NO: 5. The allosteric L-ribozyme comprises a spiegelmer specific for theophylline as the target binding moiety. The complex of the allosteric L-ribozyme and the substrate is shown on Fig. 3

The method of manufacture and performing a test for determining the impact of various concentrations of the target molecule, i.e. theophylline, on the observed fluorescence which could be used as a calibration curve in subsequent analysis for theophylline, can be summarized as follows:

300 pmol SEQ ID NO: 1 and 400 pmol SEQ ID NO: 5 in 50 µl water were denatured for 3 min at 95°C and renatured for 5 min at 37°C.

5 µl of the obtained mixture were mixed with 10 µl buffer A (1500 mM NaCI, 40 mM KCl, 100 mM Hepes-Na pH 7.4), 40 µl 50 mM MgCl₂, 10 µl of a theophylline stock (concentration 1-50 mM) and 20 µl water. The Fluorescence measurements were done at a FLUOSTAR (BMG ). Samples were analysed in a black 96 well plate (Costar) at 490 nm Excitation and 525 nm Emission wave length (40 pulse per cycle, 100 cycles a 10 sec). The time resolved fluorescence was recorded and analysed. The original data are shown in table 2.

**Table 2:**

| nmol Theophylline per sample | relative increase of Fluorescence (ΔrFU) |
|---|---|
| 0 | 65 |
| 10 | 290 |
| 50 | 1364 |
| 100 | 1589 |
| 500 | 2000 |

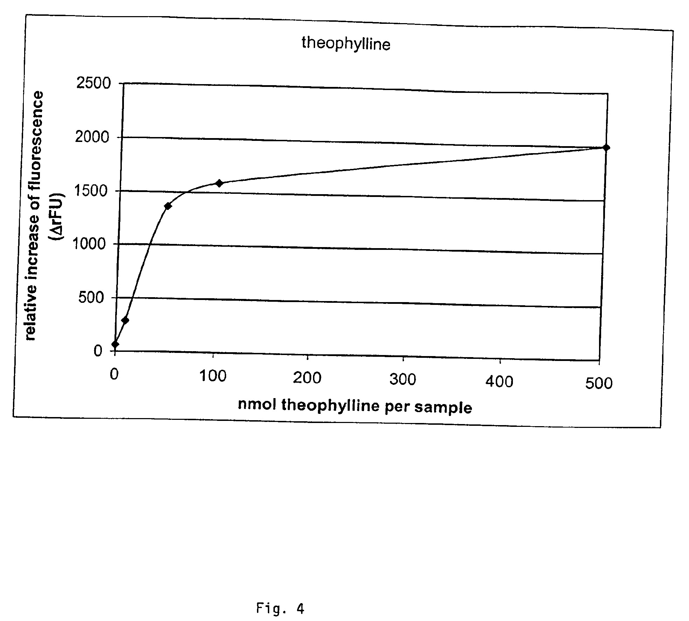

The results of this assay are also depicted in Fig. 4.

### Example 4: Allosteric ribozyme based biosensors for L-adenosine

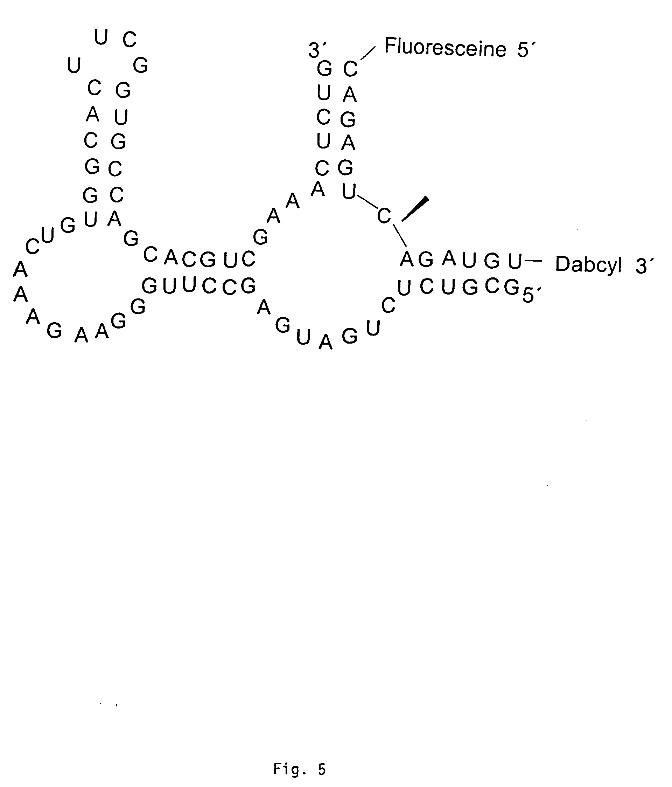

This L-adenosine specific biosensor was constructed by combining the allosteric L-ribozyme comprising a nucleic acid sequence according to SEQ ID NO: 3 and a substrate comprising a nucleic acid sequence according to SEQ ID NO: 4. The allosteric L-ribozyme comprises a spiegelmer specific for L-adenosine as the target binding moiety. The complex of the allosteric L-ribozyme and the substrate is shown on Fig. 5.

The method of manufacture and performing a test for determining the impact of various concentrations of the target molecule, i.e. L-adenosine, on the observed fluorescence which could be used as a calibration curve in subsequent analysis for theophylline, can be summarized as follows:

600 pmol SEQ ID NO: 3 and 1000 pmol SEQ ID NO: 4 in 42 µl water were denatured for 3 min at 95°C and renatured for 5 min at 37°C.

6.5 µl of the obtained mixture were mixed with 10 µl buffer A (1500 mM NaCl, 40 mM KCl, 100 mM Hepes-Na pH 7.4), 40 µl 50 mM MgCl₂, 10 µl of a L-adenosine stock solution (concentration 1-50 mM) and 20 µl water. The fluorescence measurements were done at a FLUOSTAR (BMG ). Samples were analysed in a black 96 well plate (Costar) at 490 nm Excitation and 525 nm Emission wave lenght (40 pulse per cycle, 100 cycles a 10 sec(or 50 cycles a 20 sec).

The time resolved fluorescence was recorded and analysed. The original data are shown in table 3

**Table 3:**

| nmol L-Aderiosine per sample | relative increase of Fluorescence (ΔrFU) |
|---|---|
| 0 | 904 |
| 10 | 1000 |
| 50 | 1566 |
| 100 | 2108 |
| 500 | 4096 |

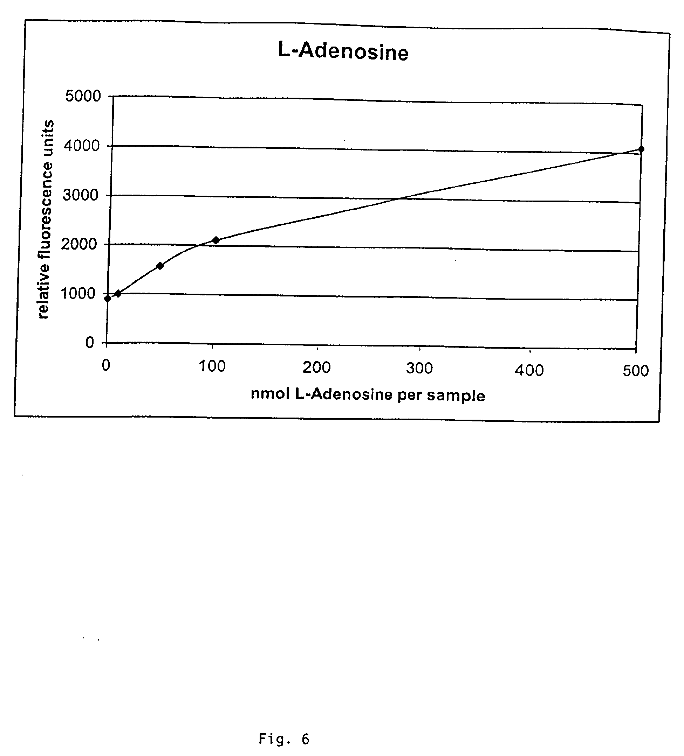

The results of this assay are also depicted in Fig. 6.

The features of the present invention disclosed in the specification, the claims and/or the drawings may both separately and in any combination thereof be material for realizing the invention in various forms thereof.

## Claims

1. An allosteric (deoxy) ribozyme, preferably a hammerhead (deoxy) ribozyme, **characterized in that** the ribozyme consists of L-nucleotides.

2. A polynucleotide comprising a (deoxy) ribozyme moiety, preferably a hammerhead ribozyme moiety, which comprises a catalytic domain and a binding site for a ribozyme substrate, and an target binding moiety, whereby the target binding moiety is specific for a target molecule, wherein the catalytic activity of the catalytic domain is reduced in the absence of the target molecule compared to the activity of the catalytic domain in the presence of the target molecule, **characterized in that** the polynucleotide consists of L-nucleotides.

3. A polynucleotide comprising a (deoxy) ribozyme moiety, preferably a hammerhead ribozyme moiety, which comprises a catalytic domain and a binding site for a ribozyme substrate, and an target binding moiety, whereby the target binding moiety is specific for a target molecule, preferably a polynucleotide according to claim 2, further comprising the target molecule bound to the target binding moiety, whereby the catalytic activity of the catalytic domain is increased in the presence of the target molecule compared to the activity of the catalytic domain in the absence of the target molecule, **characterized in that** the polynucleotide consists of L-nucleotides.

4. A polynucleotide comprising a (deoxy) ribozyme moiety, preferably a hammerhead ribozyme moiety, which comprises a catalytic domain and a binding site for a ribozyme substrate, and an target binding moiety, whereby the target binding moiety is specific for a target molecule, wherein the catalytic activity of the catalytic domain is increased in the absence of the target molecule compared to the activity of the catalytic domain in the presence of the target molecule, **characterized in that** the polynucleotide consists of L-nucleotides.

5. A polynucleotide comprising a (deoxy) ribozyme moiety, preferably a hammerhead ribozyme moiety, which comprises a catalytic domain and a binding site for a ribozyme substrate, and an target binding moiety, whereby the target binding moiety is specific for a target molecule, preferably a polynucleotide according to claim 4, further comprising the target molecule bound to the target binding moiety, whereby the catalytic activity of the catalytic domain is decreased in the presence of the target molecule of the aptamer compared to the activity of the catalytic domain in the absence of the target molecule, **characterized in that** the polynucleotide consists of L-nucleotides.

6. A polynucleotide comprising a (deoxy) ribozyme moiety, preferably a hammerhead ribozyme moiety, which comprises a catalytic domain and a binding site for a ribozyme substrate, and an target binding moiety, whereby the target binding moiety is specific for a target, more particularly a polynucleotide according to any of claims 2 to 5, wherein the base pairing pattern of at least part of the polynucleotide in the presence of and/or upon binding of the target molecule is different from the base pairing pattern of the polynucleotide in the absence of and/or non-binding of the target molecule, **characterized in that** the polynucleotide consists of L-nucleotides.

7. The Polynucleotide according to any of claims 2 to 6, further comprising a ribozyme substrate.

8. The polynucleotide according to claim 5, **characterized in that** the ribozyme substrate is a FRET-substrate.

9. The polynucleotide according to claim 7, wherein the complex of ribozyme moiety and ribozyme substrate forms a quenching system.

10. The polynucleotide according to claim 9, **characterized in that** the quenching system is formed by a fluorophor group and a quenching group.

11. The polynucleotide according to any of claims 2 to 10, **characterized in that** the polynucleotide consists of L-RNA, L-DNA or mixtures thereof.

12. A complex comprising the polynucleotide according to any of claims 1 to 11 and a ribozyme substrate, preferably a ribozyme substrate for the ribozyme moiety of the polynucleotide.

13. The complex according to claim 12, further comprising a target molecule, preferably a target molecule for the target binding moiety of the polynucleotide.

14. A composition comprising the polynucleotide according to any of claims 1 to 11 and a ribozyme substrate, preferably a ribozyme substrate for the ribozyme moiety of the polynucleotide.

15. The composition according to claim 14, further comprising a target molecule, preferably a target molecule for the target binding moiety of the polynucleotide.

16. A biosensor comprising a polynucleotide according to any of claims 1 to 11.

17. The biosensor according to claim 16, whereby the polynucleotide is immobilized to a support.

18. Method for determining the presence and/or concentration of an analyte comprising the steps of
a) providing an oligonucleotide according to any of claims 1 to 11,
b) optionally determining the catalytic activity of the ribozyme moiety,
c) providing a substrate for the ribozyme moiety of the polynucleotide and reacting such substrate with the polynucleotide,
d) optionally determining the catalytic activity of the ribozyme,
e) adding a sample presumably containing the analyte,
f) determining whether the substrate is cleaved by the ribozyme moiety,
wherein the analyte is the target molecule of the target binding moiety of the polynucleotide.

19. Method for determining the presence and/or concentration of an analyte comprising the steps of
a) providing an oligonucleotide according to any of claims 7 to 11,
b) optionally determining the catalytic activity of the ribozyme moiety,
c) adding a sample presumably containing the analyte,
d) determining whether the substrate is cleaved by the ribozyme moiety,
wherein the analyte is the target molecule of the target binding moiety of the polynucleotide.

20. The method according to claim 18 or 19, wherein the substrate comprises a fluorescent group and a quenching group and whereby after cleavage of the substrate by the catalytic domain of the ribozyme the quenching of the fluorescence is reduced.

21. Kit comprising
a) an allosteric (deoxy) ribozyme according to claim 1 and/or a polynucleotide according to any of claims 2 to 11 and, optionally,
b) a substrate for the ribozyme moiety of the polynucleotide according to any of claims 1 to 11.

22. Method for the generation of an allosteric L-(deoxy) ribozyme, preferably according to claim 1 and/or a polynucleotide according to any of claims 2 to 11, with an allosteric effector and/or a target molecule being a distinct enantiomer, comprising the following steps:
a) providing a D-polynucleotide, preferably a library of D-polynucleotides, whereby the polynucleotide comprises a (deoxy) ribozyme moiety, preferably a hammerhead ribozyme moiety, which comprises a catalytic domain, a binding site for a ribozyme substrate and a ribozyme substrate, and a candidate target binding moiety, whereby the candidate target binding moiety is of random sequence;
b) selecting for D-polynucleotide(s) which is/are not catalytically active in the absence of the optical antipode of the allosteric effector and/or of the target molecule;
c) contacting the selected D-polynucleotide(s) from step b) with the optical antipode of the allosteric effector and/or of the target molecule;
d) selecting the D-polynucleotide(s) the catalytic domain's activity of which is increased upon contacting and/or binding of the optical antipode of the allosteric effector and/or the target molecule; and
e) preparing L-polynucleotide(s) having a sequence identical to those D-polynucleotide(s) selected in step d).

23. Method for the generation of an allosteric L-(deoxy) ribozyme, preferably according to claim 1 and/or a polynucleotide according to any of claims 2 to 11, with an allosteric effector and/or a target molecule being a distinct enantiomer, comprising the following steps:
a) providing a D-polynucleotide, preferably a library of D-polynucleotides, whereby the polynucleotide comprises a (deoxy) ribozyme moiety, preferably a hammerhead ribozyme moiety, which comprises a catalytic domain, a binding site for a ribozyme substrate and a ribozyme substrate, and a candidate target binding moiety, whereby the candidate target binding moiety is of random sequence;
b) selecting for D-polynucleotide(s) which is/are catalytically active in the absence of the optical antipode of the allosteric effector and/or of the target molecule;
c) contacting the selected D-polynucleotide(s) from step b) with the optical antipode of the allosteric effector and/or of the target molecule;
d) selecting the D-polynucleotide(s) the catalytic domain's activity of which is decreased upon contacting and/or binding of the optical antipode of the allosteric effector and/or the target molecule; and
e) preparing L-polynucleotide(s) having a sequence identical to those D-polynucleotide(s) selected in step d).

24. Method for the generation of an allosteric L-(deoxy) ribozyme, preferably according to claim 1 and/or a polynucleotide according to any of claims 2 to 11, with an allosteric effector and/or a target molecule being a distinct enantiomer, comprising the following steps:
a) providing a L-polynucleotide, preferably a library of L-polynucleotides, whereby the polynucleotide comprises a (deoxy) ribozyme moiety, preferably a hammerhead (deoxy) ribozyme moiety, which comprises a catalytic domain, a binding site for a ribozyme substrate and a ribozyme substrate, and a candidate target binding moiety, whereby the candidate target binding moiety is of random sequence;
b) selecting for L-polynucleotide(s) which is/are not catalytically active in the absence of the allosteric effector and/or of the target molecule;
c) contacting the selected L-polynucleotide(s) from step b) with the allosteric effector and/or of the target molecule;
d) selecting the L-polynucleotide(s) the catalytic domain's activity of which is increased upon contacting and/or binding of the allosteric effector and/or the target molecule; and
e) preparing L-polynucleotide(s) having a sequence identical to those D-polynucleotide(s) selected in step d).

25. Method for the generation of an allosteric L-(deoxy) ribozyme, preferably according to claim 1 and/or a polynucleotide according to any of claims 2 to 11, with an allosteric effector and/or a target molecule being a distinct enantiomer, comprising the following steps:
a) providing a L-polynucleotide, preferably a library of L-polynucleotides, whereby the polynucleotide comprises a (deoxy) ribozyme moiety, preferably a hammerhead (deoxy) ribozyme moiety, which comprises a catalytic domain, a binding site for a ribozyme substrate and a ribozyme substrate, and a candidate target binding moiety, whereby the candidate target binding moiety is of random sequence;
b) selecting for L-polynucleotide(s) which is/are catalytically active in the absence of the allosteric effector and/or of the target molecule;
c) contacting the selected L-polynucleotide(s) from step b) with the allosteric effector and/or of the target molecule;
d) selecting the L-polynucleotide(s) the catalytic domain's activity of which is decreased upon contacting and/or binding of the allosteric effector and/or the target molecule; and
e) preparing L-polynucleotide(s) having a sequence identical to those D-polynucleotide(s) selected in step d).

26. Method according to claim 22 or 23, **characterized in that** the D-polynucleotids(s) is/are immobilized.

27. Method according to claim 24 or 25, **characterized in that** the L-polynucleotids(s) is/are immobilized.

28. Method according to any of claims 22 to 27, **characterized in that** the random sequence has a length of about 20 to 80 nucleotides, preferably 30 to 60 nucleotides and more preferably 40 nucleotides.

29. Method for the generation of a L-nucleic acid binding to a target molecule in a distinct enantiomeric form comprising
a) the steps a) to d) of the method according to any of claims 22, 23,26 or 28;
b) determining the target binding moiety of the polynucleotide(s) according step d) of the methods according to any of claims 22, 23, 26 or 28; and
c) preparing L-polynucleotide(s) having a sequence identical to the target binding moiety of the polynucleotide(s) determined in step b).
wherein the target molecule in the distinct enantiomeric form corresponds to the allosteric effector and/or target molecule being a distinct enantiomer.

30. Method for the generation of a L-nucleic acid binding to a target molecule in a distinct enantiomeric form comprising the steps
a) of the method according to any of claims 22, 23, 26 or 28;
b) determining the target binding moiety of the polynucleotide(s) according step e) of the methods according to any of claims 22, 23, 26 or 28;
c) preparing L-polynucleotide(s) having a sequence identical to the target binding moiety of the polynucleotide(s) determined in step b).
wherein the target molecule in the distinct enantiomeric form corresponds to the allosteric effector and/or target molecule being a distinct enantiomer.

31. Method for the generation of a L-nucleic acid binding to a target molecule in a distinct enantiomeric form comprising the steps
a) of the method according to any of claims 24, 25, 27 or 28;
b) determining the target binding moiety of the polynucleotide(s) according step d) of the methods according to any of claims 24, 25, 27 or 28;
c) preparing L-polynucleotide(s) having a sequence identical to the target binding moiety of the polynucleotide(s) determined in step b).
wherein the target molecule in the distinct enantiomeric form corresponds to the allosteric effector and/or target molecule being a distinct enantiomer.

32. Method according to any of claims 22 to 31, **characterized in that** the target molecule in the distinct enantiomeric form and/or the allosteric effector in the distinct enantiomeric form is the naturally occurring form of the target molecule and/or of the allosteric effector.
